# EUROPÄISCHE PATENTSCHRIFT

(11) **EP 3 432 855 B1**
(45) Veröffentlichungstag und Bekanntmachung des Hinweises auf die Patenterteilung: **14.10.2020**
(21) Anmeldenummer: 17709685.6
(22) Anmeldetag: 09.03.2017
(51) Int. Cl.: A61K 8/26, A61K 8/49, A61Q 15/00, A61K 8/06, A61K 8/86, A61K 8/39, A61K 8/02, A61K 8/34, A61K 8/36, A61K 8/37, A61K 8/92

(54) **ANTITRANSPIRANTSTIFT IN FORM EINER O/W-EMULSION**
ANTIPERSPIRANT STICK IN THE FORM OF AN O/W EMULSION
STICK DEODORANT SOUS LA FORME D'UNE EMULSION H/E

(30) Priorität: 22.03.2016 DE 102016204685
(43) Veröffentlichungstag der Anmeldung: 30.01.2019
(73) Patentinhaber: Beiersdorf AG, 20253 Hamburg (DE)
(72) Erfinder: OELRICHS, Ilka, 25436 Tornesch (DE); SCHECKER, Franziska, 21244 Buchholz (DE); HALLMANN, Marita, 22417 Hamburg (DE)
(86) Internationale Anmeldenummer: PCT/EP2017/055562
(87) Internationale Veröffentlichungsnummer: WO 2017/162443

(56) Entgegenhaltungen:
- EP-A1- 2 032 118
- WO-A2-01/89452
- WO-A2-2012/080017
- DE-A1-102010 044 787
- US-A1- 2007 020 213

## Beschreibung

Die vorliegenden Erfindung betrifft eine kosmetische Zubereitung in Form einer O/W-Emulsion mit verbesserter Hautverträglichkeit, umfassend kurzkettige Alkan-1,2-diole, Emulgatoren, Fettalkohole, Öle, Wachse, Carbonsäuren, antitranspirant Wirkstoff und Wasser, wobei die Zubereitung dadurch gekennzeichnet ist, dass diese fest und formstabil ist.

Antitranspirantien (AT) oder Deodorantien (Deo) dienen dazu, Körpergeruch zu beseitigen oder zu verhindern, der entsteht, wenn der an sich geruchlose frische Schweiß durch Mikroorganismen zersetzt wird.

Im allgemeinen Sprachgebrauch erfolgt nicht immer eine klare Trennung der Begriffe "Deodorant" und "Antitranspirant". Vielmehr werden - insbesondere auch im deutschsprachigen Raum - Produkte zur Anwendung im Achselbereich pauschal als Deodorantien bzw. "Deos" bezeichnet. Dies geschieht unbeachtlich der Frage, ob auch eine antitranspirante Wirkung vorliegt.

Antitranspirantien (AT) sind Mittel, die die Absonderung von Schweiß (Transpiration) durch Blockierung der Schweißdrüsenausgänge behindern sollen. In den weitaus meisten Antitranspirantien kann durch Adstringentien - vorwiegend Aluminiumsalze wie Aluminiumhydroxychlorid (Aluchlorhydrat, ACH), Aluminiumchlorid oder Komplexe aus Aluminium Zirconium Tetrachlorohydrate und Glycin - die Bildung des Schweißes reduziert werden. Neben einer antitranspiranten bzw. schweißreduzierenden Wirkung zeigen Aluminiumverbindungen ebenfalls eine antimikrobielle Wirkung und sind somit auch Deodorantien.

Zur Applikation auf die Haut werden antitranspirant wirkende Wirkstoffe in kosmetische Zubereitungen eingearbeitet. Eine beim Verbraucher beliebte Art von Antitranspirantien zur Applikation im Achselbereich, sind feste, antitranspirant wirkende kosmetische Zubereitungen, welche oftmals als Antitranspirantstifte bezeichnet werden. Bei der Applikation der Antitranspirantstifte wird die feste Zubereitung durch Abrieb an die Haut übertragen.

Heute sind Antitranspirantien Köperpflegeprodukte des täglichen Lebens. Sie werden vom Verbraucher meist nicht nur morgens aufgetragen, sondern auch nach Sportaktivitäten mit anschließender Dusche. Das tägliche Mitführen der Antitranspirantien als persönliche Gegenstände ist somit gängige Praxis. Dabei werden die Produkte teilweise extremen Temperaturen ausgesetzt. Ein Aufheizen durch Sonneneinstrahlung auf Temperaturen um 50°C ist im Sommer in Süd- und Mitteleuropa sowie ganzjährig in äquatornahen Regionen möglich. Im Winter in Mitteleuropa und in kälteren Erdregionen sind die Produkte hingegen Temperaturen unter dem Gefrierpunkt ausgesetzt. Ferner können die Produkte gerade im Sommer starken Temperaturschwankungen zwischen klimatisierten Gebäuden und Außenluft ausgesetzt sein. Unter diesen Bedingungen kommt es oftmals zu Verformungen oder Ausfrierenden mit anschließender Rissbildung, welche vom Verbraucher als unästhetisch beschrieben werden. Nachteilig für den Hersteller ist dabei, dass die Wiederverkaufsrate der Produkte sinkt.

Aus WO 9959537 A1 sind kosmetische Stifte bekannt, die bereits ab einer Temperatur von 30°C schmelzen und so eine unzureichende Formstabilität aufweisen.

Des Weiteren sind aus DE 19962878 A1 wasserhaltige Antitranspirant-Zusammensetzungen bekannt, die einen Wassergehalt von bis zu 70 Gew.-% aufweisen. Vorteilhaft an dem hohen Wassergehalt der Antitranspirant-Zusammensetzungen ist, dass bei Auftragung der Zusammensetzung auf die Haut ein frisches, kühlendes Gefühl wahrgenommen wird. Nachteilig an den Antitranspirant-Zusammensetzungen ist jedoch, dass die Antitranspirant-Zusammensetzungen bevorzugt eine Viskosität von 200000-1500000 mPa s bei 21°C aufweisen. Demnach sind die Antitranspirant-Zusammensetzungen als Creme zu bezeichnen. Nachteilig an Cremes ist, dass diese nicht fest und formstabil sind und so zumeist mit den Händen oder einem Hilfsmittel, wie einem Tuch oder einem Pad, unter den Achseln aufgetragen werden. Eine Anwendung von festen Antitranspirantstiften ist hingegen praktischer und vom Verbraucher bevorzugt, da diese direkt aus dem Packmittel heraus appliziert werden können.

WO 2006012971 A1 offenbart antitranspirant wirksame kosmetische Zubereitungen auf Basis einer O/W-Emulsion, welche kurzkettigen Alkan-1,2-diole mit einer Alkylkette mit 3 bis 5 C-Atomen in einem Anteil von 4 Gew.-% bis 25 Gew.-%, bezogen auf das Gesamtgewicht der Zubereitung, enthalten. Feste kosmetische Formulierungen werden ausschließlich erhalten, wenn der Gewichtsanteil von Alkan-1,2-diolen mit einer Alkylkette mit 3 bis 5 C-Atomen mindestens 12 Gew.-%, bezogen auf das Gesamtgewicht der Zubereitung, beträgt.

Die Einarbeitung von Alkan-1,2-diolen mit einer Alkylkette mit 3 bis 5 Kohlenstoffatomen ist eine gebräuchliche Methode zur Erniedrigung des Gefrierpunkts und somit der Verhinderung von Ausfrierungen von Antitranspirantstiften, die als Öl-in-Wasser Emulsion (O/W-Emulsion) formuliert sind.

Nachteilig ist jedoch, dass Alkan-1,2-diolen, wie beispielsweise Propan-1,2-diol, hautirritierend wirken können. So weist Propan-1,2-diol bei Einsatz in von Konzentrationen oberhalb von 50 Gew.-%, bezogen auf das Gesamtgewicht der Zubereitung, eine starke hautirritierende Wirkung auf (A. Scheman, S. Jacob, M. Zirwas et al., Dis. Mon. 54, 7-156 (2008)). Zwar nimmt die hautirritierende Wirkung von Propan-1,2-diol mit abnehmender Konzentration ab, dennoch sind Studien bekannt, die bei Epikutantests mit 10 Gew.-% Propan-1,2-diol bei 4% der Teilnehmer allergische Hautreaktionen nachweisen konnten (Propylene Glycol Information Update, American Chemistry Council's Propylene Oxide/Propylene Glycol Panel, 2001). Des Weiteren wird in der Veröffentlichung Antitranspirant and Deodorant Allergy - Diagnosis and Management (M. J. Zirwas, J. Moennich, Journal of clinical and Aesthetic Dermatology, Vol. 1, Nr. 3 (2008)) darauf hingewiesen, dass Propan-1,2-diol eines der am häufigsten vorkommenden Allergene in Antitranspirantien und Deodorantien ist. Selbst bei Propan-1,2-diol Konzentrationen von 3 Gew.-% bis 5 Gew.-% kann eine allergische Reaktion von Anwendern mit Propan-1,2-diol Allergie nicht ausgeschlossen werden.

Aufgabe der vorliegenden Erfindung war es eine antitranspirant-wirksame kosmetische Zubereitungen in Form einer O/W-Emulsion bereitzustellen, die die Nachteile des Standes der Technik nicht aufweist. Insbesondere sollte die kosmetische Zubereitung fest und formstabil sein und gleichzeitig eine verbesserte Hautverträglichkeit und/oder ein geringes allergenes Potential aufweisen.

Überraschend wurden die vorstehenden Aufgaben durch die vorliegende Erfindung gelöst.

Gegenstand der vorliegenden Erfindung ist eine kosmetische Zubereitung in Form einer O/W-Emulsion, umfassend
a) 0,1 Gew.-% bis 2,5 Gew.-% ein oder mehrere Alkan-1,2-diole mit einer Alkylkette mit 3 bis 5 C-Atomen,
b) zwei oder mehr Emulgatoren, wobei mindestens ein O/W-Emulgator mit einem HLB-Wert größer 8 und mindestens ein W/O-Emulgator mit einem HLB-Wert bis maximal 8 enthalten ist,
c) ein oder mehrere Fettalkohole, welche ausgewählt sind aus aliphatischen, linearen, primären Alkanolen mit einer Kettenlänge von 6 bis 22 C-Atomen,
d) ein oder mehrere bei 20°C und 1013 hPa flüssige Öle,
e) ein oder mehrere Wachse, die ab einer Temperatur von 40°C bei 1013 hPa ohne Zersetzung schmelzen,
f) eine oder mehrere gesättigte und/oder ungesättigte lineare Carbonsäuren mit einer Kettenlänge von 10 bis 20 C-Atomen,
g) einen oder mehrere antitranspirant wirkende Wirkstoffe, und
h) Wasser,
jeweils bezogen auf das Gesamtgewicht der Zubereitung, wobei die kosmetische Zubereitung einen Penetrationskraftwert von 300 Gramm-Kraft bis 600 Gramm-Kraft aufweist.

Die antitranspirant wirksame kosmetische Zubereitung der vorliegenden Erfindung weist nicht nur eine verbesserte Hautverträglichkeit und/oder ein geringes allergenes Potential auf, sondern ist gleichzeitig fest und formstabil.

Auch Gegenstand der Erfindung ist ein Verfahren zum Verringern oder Hemmen von Transpiration, wobei das Verfahren das Anwenden einer gegen Transpiration wirksamen Menge der erfindungsgemäßen kosmetischen Zubereitung auf die Haut umfasst.

Auch Gegenstand der Erfindung ist die Verwendung der erfindungsgemäßen kosmetischen Zubereitung auf der Haut zur Verringerung von Transpiration.

Da Wasser in der erfindungsgemäßen Zubereitung die äußere Phase bildet, können nach Anwendung auf der Haut verbleibende Rückstände mit Wasser leicht abgespült werden. Rückstände auf der Kleidung können ebenfalls mit Wasser leicht entfernt werden.

Alle nachstehenden Gewichtsprozentangaben (Gew.-%) beziehen sich, sofern nicht anders angegeben auf das Gesamtgewicht der erfindungsgemäßen Zubereitung.

Die erfindungsgemäße kosmetische Zubereitung enthält ein oder mehrere Alkan-1,2-diole mit einer Alkylkette mit 3 bis 5 C-Atomen in einem Gesamtanteil von 0,1 Gew.-% bis 2,5 Gew.-%, bevorzugt 0,2 Gew.-% bis 2 Gew.-%.

Es ist erfindungsgemäß bevorzugt, wenn als Alkan-1,2-diol mit einer Alkylkette mit 3 bis 5 C-Atomen Propan-1,2-diol in der erfindungsgemäßen kosmetischen Zubereitung enthalten ist.

Es ist insbesondere bevorzugt, wenn neben Propan-1,2-diol keine weiteren Alkan-1,2-diole mit einer Alkylkette mit 3 bis 5 C-Atomen in der kosmetischen Zubereitung enthalten sind. Da die ein oder mehreren Alkan-1,2-diole sich höchst wahrscheinlich in der Wasserphase der O/W-Emulsion befinden, geht eine Einarbeitung in die Emulsion mit einer Erniedrigung des Gefrierpunkts einher. Da Alkan-1,2-diole allerdings auch Lösungsmittel sind, erwartet der Fachmann, dass sich bei Zugabe die Formstabilität der Emulsion besonders bei höheren Temperaturen verschlechtert und eine cremförmige Lösung erhalten wird. Überraschenderweise wurde gefunden, dass die erfindungsgemäße kosmetische Zubereitung formstabil ist.

Emulgatoren setzen im Allgemeinen die Grenzflächenspannung zwischen Öl- und Wasserphasen herab und erreichen neben der Verringerung der Grenzflächenarbeit auch eine Stabilisierung der gebildeten Emulsion. Sie stabilisieren die gebildete Emulsion durch Grenzflächenfilme sowie durch Ausbildung sterischer oder elektrischer Barrieren, wodurch das Zusammenfließen (Koaleszenz) der emulgierten Teilchen verhindert wird.

Damit Verbindungen als Emulgatoren wirksam sein können, müssen sie eine bestimmte Molekülstruktur aufweisen. Strukturelles Kennzeichen solcher Verbindungen ist ihr amphiphiler Molekülaufbau. Das Molekül einer solchen Verbindung besitzt wenigstens eine Gruppe mit Affinität zu Substanzen starker Polarität (polare Gruppe) und wenigstens eine Gruppe mit Affinität zu unpolaren Substanzen (apolare Gruppe).

Es wird dabei unterschieden zwischen nicht-ionischen, anionischen und kationischen Emulgatoren. Ein Kennzeichen für die Hydrophilie eines gegebenen Emulgators ist dessen HLB-Wert, der sich nach folgender Formel ergibt: HLB = 20 x (1- Mₗᵢₚₒₚₕᵢₗ/M), wobei Mₗᵢₚₒₚₕᵢₗ für die Molmasse des lipophilen Anteils im Emulgator und M für die Molmasse des gesamten Emulgators steht.

Im Allgemeinen gelten Emulgatoren mit einem HLB-Wert bis maximal 8 als W/O-Emulgatoren. O/W-Emulgatoren hingegen weisen HLB-Werte von größer 8 auf.

Die erfindungsgemäße Zubereitung umfasst zwei oder mehre Emulgatoren, wobei mindestens ein O/W-Emulgator mit einem HLB-Wert größer 8 und mindestens ein W/O-Emulgator mit einem HLB-Wert bis maximal 8 enthalten ist.

Vorteilhaft beträgt der Gesamtanteil der Emulgatoren 1,5 Gew.-% bis 5 Gew.-%, bevorzugt 2 Gew.-% bis 3 Gew.-%.

Die erfindungsgemäßen Emulgatoren können gewählt werden aus der Gruppe der anionischen, kationischen, amphoteren und nicht-ionischen Emulgatoren. Erfindungsgemäß bevorzugt ist der Einsatz von nicht-ionischen Emulgatoren.

Erfindungsgemäße nicht ionische Emulgatoren sind beispielsweise Propylene Glycol Isostearate (HLB 2.5), Glycol Stearate (HLB 2.9), Glyceryl Isostearate (HLB 3,5), Sorbitan Sesquioleate (HLB 3.7), Glyceryl Stearate (HLB 3.8), Lecithin (HLB 4), Sorbitan Oleate (HLB 4.3), Sorbitan Monostearate NF (HLB 4.7), Sorbitan Stearate (HLB 4.7), Sorbitan Isostearate (HLB 4.7), Steareth-2 (HLB 4.9), Oleth-2 (HLB 4.9), Glyceryl Laurate (HLB 5.2), Ceteth-2 (HLB 5.3), PEG-30 Dipolyhydroxystearate (HLB 5.5), Glyceryl Stearate SE (HLB 5.8), Sorbitan Stearate (and) Sucrose Cocoate (HLB 6), PEG-4 Dilaurate (HLB 6), PEG-8 Dioleate (HLB 8), Sorbitan Laurate (HLB 8.6), PEG-40 Sorbitan Peroleate (HLB 9), Laureth-4 (HLB 9.7), PEG-7 Glyceryl Cocoate (HLB 10), PEG-20 Almond Glycerides (HLB 10), PEG-25 Hydrogenated Castor Oil (HLB 10.8), Stearamide MEA (HLB 11), Glyceryl Stearate (and) PEG-100 Stearate (HLB 11), Polysorbate 85 (HLB 11), PEG-7 Olivate (HLB 11), Cetearyl Glucoside (HLB 11), PEG-8 Oleate (HLB 11.6), Polyglyceryl-3 Methyglucose Distearate (HLB 12), PG-10 Stearate (HLB 12), Oleth-10 (HLB 12.4), Oleth-10 / Polyoxyl 10 Oleyl Ether NF (HLB 12.4), Ceteth-10 (HLB 12.9), PEG-8 Laurate (HLB 13), Ceteareth-12 (HLB 13,5), Cocamide MEA (HLB 13.5), Polysorbate 60 NF (HLB 14.9), Polysorbate 60 (HLB 14.9), PEG-40 Hydrogenated Castor Oil (HLB 15), Polysorbate 80 (HLB 15), Isosteareth-20 (HLB 15), PEG-60 Almond Glycerides (HLB 15), Polysorbate 80 NF (HLB 15), PEG-150 Laurate, PEG-20 Methyl Glucose Sesquistearate (HLB 15), Ceteareth-20 (HLB 15.2), Oleth-20 (HLB 15.3), Steareth-20 (HLB 15.3), Steareth-21 (HLB 15.5),Ceteth-20 (HLB 15.7), Isoceteth-20 (HLB 15.7), PEG-30 Glyceryl Laurate (HLB 16), Polysorbate 20 (HLB 16.7), Polysorbate 20 NF (HLB 16.7), Laureth-23 (HLB 16.9), PEG-100 Stearate (HLB 18.8), Steareth-100 (HLB 18.8), PEG-80 Sorbitan Laurate (HLB 19.1).

Erfindungsgemäß bevorzugte nicht-ionische O/W-Emulgatoren weisen einen HLB-Wert größer 8, besonders bevorzugt einen HLB-Wert zwischen 13 und 18, ganz besonders bevorzugt einen HLB-Wert zwischen 14,5 bis 16,5 auf.

In einer weiteren erfindungsgemäßen Ausführungsform werden bevorzugt nicht ionische O/W-Emulgatoren ausgewählt, die Anlagerungsprodukten von 15 bis 25 Ethylenoxid-Einheiten an gesättigte oder ungesättigte oder teilweise gesättigte Alkanole mit einer Kettenlänge von 12 bis 20 C-Atomen sind, wobei insbesondere Anlagerungsprodukten von 20 bis 21 Ethylenoxid-Einheiten an gesättigte oder ungesättigte oder teilweise gesättigte Alkanole mit einer Kettenlänge von 18 C-Atomen bevorzugt sind.

In einer weiteren besonders bevorzugten erfindungsgemäßen Ausführungsform werden nicht ionische O/W-Emulgatoren ausgewählt, die einen HLB-Wert zwischen 14,5 und 16,5 haben und Anlagerungsprodukte von 20 bis 21 Ethylenoxid-Einheiten an gesättigte oder ungesättigte oder teilweise gesättigte Alkanole mit einer Kettenlänge von 18 C-Atomen sind. Erfindungsgemäß bevorzugte nicht ionische W/O-Emulgatoren weisen einen HLB-Wert bis maximal 8, besonders bevorzugt einen HLB-Wert zwischen 3 und 7, ganz besonders bevorzugt einen HLB-Wert zwischen 4 und 6 auf.

In einer weiteren erfindungsgemäßen Ausführungsform werden bevorzugt nicht ionische W/O-Emulgatoren ausgewählt, die Anlagerungsprodukten von 1 bis 4 Ethylenoxid-Einheiten an gesättigte oder ungesättigte oder teilweise gesättigte Alkanole mit einer Kettenlänge von 12 bis 20 C-Atomen sind, wobei insbesondere Anlagerungsprodukten von 2 bis 3 Ethylenoxid-Einheiten an gesättigte oder ungesättigte oder teilweise gesättigte Alkanole mit einer Kettenlänge von 18 C-Atomen bevorzugt sind.

In einer weiteren besonders bevorzugten erfindungsgemäßen Ausführungsform werden nicht ionische W/O-Emulgatoren ausgewählt, die einen HLB-Wert zwischen 14,5 und 16,5 haben und Anlagerungsprodukte von 2 bis 3 Ethylenoxid-Einheiten an gesättigte oder ungesättigte oder teilweise gesättigte Alkanole mit einer Kettenlänge von 18 C-Atomen sind.

Bevorzugt ist der Einsatz von zwei nicht ionischen O/W-Emulgatoren und eines nicht ionischen W/O-Emulgators.

Ferner ist es bevorzugt, wenn die jeweiligen Emulgatoren einzeln in einem Anteil von 0,5 Gew.-% bis 1 Gew.-% enthalten sind.

Erfindungsgemäße Mischungen aus nicht ionische W/O- und O/W-Emulgator sind bevorzugt zu wählen aus Steareth-2 als W/O-Emulgator und Oleth-20 und Steareth-21 als O/W-Emulgatoren.

Es ist insbesondere vorteilhaft im Sinne der Erfindung, wenn neben Steareth-2, Oleth-20 und Steareth-21 keine weiteren Emulgatoren enthalten sind.

Erfindungsgemäß ist weiterhin, dass ein oder mehrere Fettalkohole in der kosmetischen Zubereitung enthalten sind. Die nachstehenden Fettalkohole werden im Sinne dieser Erfindung keinesfalls als Emulgatoren, Öle oder Wachse bezeichnet. Fettalkohole im Sinne dieser Erfindung sind ausschließlich aliphatische, lineare, primäre Alkanole mit einer Kettenlänge von 6 bis 22 C-Atomen.

Erfindungsgemäße Fettalkohole werden bevorzugt gewählt aus der Gruppe Laurylalkohol, Myristylalkohol, Cetylalkohol, Stearylalkohol, Margarylalkohol, Arachidylalhohol und Behenylalkohol.

Unter diesen werden besonders bevorzugt Cetylalkohol, Stearylalkohol und/oder Mischungen aus diesen gewählt. Mischungen aus Cetylalkohol und Stearylalkohol sind unter dem Trivialnamen Cetylstearylalkohol bekannt. Eine solche Mischung kann unter anderem unter dem Handelsnamen Kolliwax CSA50 von der Fa. BASF oder unter dem Namen Nafol 1618-JA von der Fa. Sasol bezogen werden.

Es ist insbesondere vorteilhaft im Sinne der vorliegenden Erfindung, wenn neben Cetylalkohol, Stearylalkohol und/oder Cetylstearylalkohol keine weiteren Fettalkohole in der erfindungsgemäßen kosmetischen Zubereitung enthalten sind.

Die erfindungsgemäßen Fettalkohole sind vorteilhaft in einem Gesamtanteil von 0,5 Gew.-% bis 5 Gew.-%, bevorzugt 0,75 Gew.-% bis 3,1 Gew.-% in der erfindungsgemäßen kosmetischen Zubereitung enthalten.

Ferner enthält die erfindungsgemäße kosmetische Zubereitung ein oder mehrere bei 20°C und 1013 hPa flüssige Öle.

Die nachfolgend aufgeführten Stoffgruppen und Chemikalien innerhalb der spezifizierten Eigenschaften sind im Sinne dieser Erfindung ausschließlich als Öl zu bezeichnen. Keinesfalls werden diese als Wachse oder Emulgatoren bezeichnet.

Parfümöle und ätherische Öle sind im Sinne der vorliegenden Erfindung keineswegs als Öl zu bezeichnen. Parfümöle und ätherische Öle werden stattdessen im Sinne der vorliegenden Erfindung als Parfüm bezeichnet.

Es ist Vorteilhaft im Sinne der vorliegenden Erfindung, wenn der Anteil der ein oder mehreren bei 20°C und 1013 hPa flüssigen Öle in der erfindungsgemäßen kosmetischen Zubereitung 18 Gew.-% bis 27 Gew.-%, bevorzugt 20 Gew.-% bis 25 Gew.-%, insbesondere bevorzugt 21 Gew.-% bis 24 Gew.-% beträgt.

Es hat sich überraschend gezeigt, dass eine erfindungsgemäße kosmetische Zubereitung mit ein oder mehreren bei 20°C und 1013 hPa flüssigen Öle in einem Anteil von 21 Gew.-% bis 24 Gew.-% beim Anwendung als Antitranspirantstift auf der Haut beim Verbraucher ein angenehmes, geschmeidiges Gefühl hinterlassen.

Die erfindungsgemäße Öle sind ausgewählt aus den Anlagerungsprodukten von 6-60 Propylenoxid-Einheiten an ein- oder mehrwertige Alkanole mit einer Kettenlänge von 3 bis 22 C-Atomen. Diese werden im Sinne der Erfindung als Etheröle bezeichnet. Erfindungsgemäße Beispiele dieser Etheröle sind PPG-9 Butylether, PPG-12 Butylether, PPG-14 Butylether, PPG-15 Butylether, PPG-16 Butylether, PPG-17 Butylether, PPG-18 Butylether, PPG-2 Butylether, PPG-20 Butylether, PPG-22 Butylether, PPG-24 Butylether, PPG-26 Butylether, PPG-30 Butylether, PPG-33 Butylether, PPG-40 Butylether, PPG-52 Butylether, PPG-53 Butylether und PPG-15 Stearylether, sofern diese bei 20°C und 1013 hPa flüssig sind.

Weitere erfindungsgemäße Öle können ausgewählt werden aus der Gruppe der Ester aus gesättigten und/oder ungesättigten, verzweigten und/oder unverzweigten Alkancarbonsäuren einer Kettenlänge von 3 bis 17 C-Atomen und ungesättigten und/oder ungesättigten, verzweigten und/oder unverzweigten Alkoholen einer Kettenlänge von 3 bis 10 C-Atomen sowie aus der Gruppe der Ester aus aromatischen Carbonsäuren und ungesättigten und/oder ungesättigten, verzweigten und/oder unverzweigten Alkoholen einer Kettenlänge von 3 bis 15 C-Atomen, sofern das gewählte Esteröl bei 20°C flüssig ist. Solche Esteröle können vorteilhaft gewählt werden aus der Gruppe Isopropylmyristat, Isopropylpalmitat, Isopropylstearat, Isopropyloleat, 2-Ethylhexylpalmitat, 2-Ethylhexyllaurat, C12-15 Alkylbenzoat, sowie synthetische, halbsynthetische und natürliche Gemische solcher Ester, sofern das gewählte Esteröl bei 20°C und 1013 hPa flüssig ist.

Erfindungsgemäß ist es bevorzugt eine Mischung aus drei Ölen einzusetzen, wobei zwei Öle aus der Gruppe der Etheröle und ein Öl aus der Gruppe der Esteröle gewählt werden, und alle eingesetzten Öle bei 20°C und 1013 hPa flüssig sind.

Erfindungsgemäß beträgt das Gewichtsverhältnis der Etheröle zum Esteröl in der kosmetischen Zubereitung 4:1 bis 1:1, bevorzugt 3:1 bis 2:1.

In einer erfindungsgemäßen vorteilhaften Ausführungsform umfassen die Ölmischungen zwei Etheröle und ein Esteröle, wobei als Esteröl C12-15 Alkylbenzoat enthalten ist.

In einer weiteren erfindungsgemäßen vorteilhaften Ausführungsform umfassen die Ölmischungen zwei Etheröle und ein Esteröle, wobei als Etheröle PPG-14 Butylether und PPG-15 Stearylether enthalten sind.

In einer besonders vorteilhaften erfindungsgemäßen Ausführungsform umfasst die Ölmischungen zwei Etheröle und ein Esteröl, wobei als Etheröle PPG-14 Butylether und PPG-15 Stearylether und als Esteröl C12-15 Alkylbenzoat gewählt werden.

In einer ganz besonders vorteilhaften Ausführungsform umfasst die Ölmischung 22 Gew.-% bis 28 Gew.-% PPG-14 Butylether, 40 Gew.-% bis 51 Gew.-% PPG-15 Stearylether und 23 Gew.-% bis 33 Gew.-% C12-15 Alkylbenzoat, bezogen auf das Gesamtgewicht der Ölmischung.

Es ist insbesondere vorteilhaft im Sinne der Erfindung, wenn neben PPG-14 Butylether, PPG-15 Stearylether und C12-15 Alkylbenzoat keine weiteren bei 20°C und 1013 hPa flüssigen Öle in der erfindungsgemäßen Zubereitung enthalten sind.

Die erfindungsgemäßen kosmetische Zubereitung umfasst ein oder mehrere Wachse, die ab einer Temperatur von 40°C bei 1013 hPa ohne Zersetzung schmelzen.

Erfindungsgemäße Wachse sind pflanzliche und/oder tierische Wachse, wie beispielsweise Carnaubawachs (Copernica Cerifera Cera), Reiswachs, Candelillawachs, Sonnenblumenwachs, Bienenwachs, Walrat, Korkwachs, Montanwachs und Baumwollwachs.

Weitere erfindungsgemäße Wachse sind ausgewählt aus der Gruppe der Ester, die mittels Veresterung von einer linearen oder verzweigten, gesättigten, teilweise gesättigten oder ungesättigten Carbonsäure mit einer Kettenlänge von 15 bis 36 C-Atomen und einem linearen oder verzweigten, gesättigten, teilweise gesättigten oder ungesättigten Alkanol mit einer Kettenlänge von 15 bis 36 C-Atomen hergestellt werden. Beispiele erfindungsgemäßer Esterwachse sind Cetylpalmitat und Myristylpalmitat.

Weitere erfindungsgemäße Wachse sind Triglyceride, die durch Hydrierung von Doppelbindungen ungesättigter Fettsäurereste gehärtet wurden. Beispiele dieser erfindungsgemäßen Wachse sind hydriertes Rizinusöl, hydriertes Palmöl und hydriertes Kokosöl.

Die erfindungsgemäße kosmetische Zubereitung umfassen ein oder mehrere Wachse, bevorzugt zwei Wachse, wovon ein Wachs eine Schmelztemperatur bei 1013 hPa zwischen 40°C und 50°C aufweist und ein Wachse eine Schmelztemperatur bei 1013 hPa von mehr als 50°C aufweisen. Vorteilhaft an der vorstehenden Auswahl ist, dass die resultierenden kosmetischen Zubereitungen Formstabilität und/oder eine verbesserte Festigkeit aufweisen und gleichzeitig vom Verbraucher als geschmeidig empfunden werden.

Eine erfindungsgemäß bevorzugte Wachskombination umfasst hydriertes Rizinusöl und Cetylpalmitat.

Es ist besonders bevorzugt, wenn neben hydriertem Rizinusöl und Cetylpalmitat keine weiteren Wachse in den erfindungsgemäßen kosmetischen Zubereitungen enthalten sind.

Vorteilhaft beträgt der Gesamtanteil der ein oder mehreren Wachsen, die ab einer Temperatur von 40°C bei 1013 hPa ohne Zersetzung schmelzen, in den kosmetischen Zubereitungen beträgt 8 Gew.-% bis 15 Gew.-%, bevorzugt 10 Gew.-% bis 13 Gew.-%, insbesondere bevorzugt 11 Gew.-% bis 12 Gew.-%.

Ferner enthalten die erfindungsgemäßen kosmetischen Zubereitungen eine oder mehrere gesättigte und/oder ungesättigte lineare Carbonsäuren mit einer Kettenlänge von 10 bis 20 C-Atomen. Diese Carbonsäuren werden im Sinne der Erfindung keinesfalls als Emulgator, Öl oder Wachs bezeichnet.

Die erfindungsgemäßen Carbonsäuren werden unter anderem gewählt aus der Gruppe Caprinsäure, Laurinsäure, Myristinsäure, Palmitinsäure, Stearinsäure, Ölsäure, und Arachinsäure.

Bevorzugt werden Mischungen aus zwei oder mehr erfindungsgemäßen Carbonsäuren eingesetzt.

Ein Beispiel einer solchen erfindungsgemäß besonders bevorzugten Mischung umfasst Myristinsäure, Palmitinsäure, Stearinsäure, Arachinsäure und Ölsäure. Die vorstehende Mischung an Carbonsäure ist kommerziell unter dem Handelsnamen Omyacid 48 (cosmed Grade) von der Fa. Omya Peralta erhältlich.

Es ist erfindungsgemäß vorteilhaft, wenn die ein oder mehreren gesättigten und/oder ungesättigten linearen Carbonsäuren mit einer Kettenlänge von 10 bis 20 C-Atomen in einem Gesamtanteil von 2,0 Gew.-% bis 5,0 Gew.-% und insbesondere bevorzugt in einem Gesamtanteil von 2,5 Gew.-% bis 4,5 Gew.-% in der erfindungsgemäßen kosmetischen Zubereitung enthalten sind.

Der klassische Wirkmechanismus von antitranspirant wirkenden Wirkstoffen, beruht vorwiegend auf einer Verstopfung des Schweißdrüsenausführgangs der ekkrinen Schweißdrüse, u.a. durch Verkleben von abgeschilferten Korneozyten des verhornten Ausführganges.

Die erfindungsgemäßen kosmetischen Zubereitungen enthalten einen oder mehrere antitranspirant wirkende Wirkstoff.

In einer vorteilhaften Ausführungsform der Erfindung sind als antitranspirant wirkende Wirkstoffe ein oder mehrere Aluminium- und/oder Aluminium/Zirkoniumsalze enthalten. Diese sind vorteilhaft in einem Anteil von 12,5 Gew.-% bis 25 Gew.-%, insbesondere bevorzugt in einem Anteil von 15 Gew.-% bis 20 Gew.-% in der erfindungsgemäßen kosmetischen Zubereitung enthalten.

Die aufgeführten Gewichtsprozentangeben sind Angaben die sich auf den Aktivgehalt der Wirkstoffe beziehen. Das heißt bei den Aluminium-Verbindungen auf wasserfreie Komplexe und bei den Aluminium/Zirkonium-Verbindungen auf wasser- und puffer-freie Komplexe. Als Puffer der Aluminium/Zirkonium-Verbindungen wird üblicher Weise Glycin eingesetzt.

Erfindungsgemäße Aluminiumsalze sind:
- Aluminium-Salze wie Aluminiumchlorid AlCl₃, Aluminiumsulfat Al₂(SO₄)₃
- Aluminiumchloride der empirischen Summenformel [Al₂(OH)ₘClₙ], wobei m+n=6
- Aluminiumchlorhydrat [Al₂(OH)₅Cl] x H₂O (ACH)
   ∘ Standard Al-Komplexe: Locron P (Clariant), Locron L (Clariant), Micro-Dry (Reheis), ACH-331 (Summit), Aloxicoll PF 40 (Giulini).
   ∘ Aktivierte Al-Komplexe: Reach 501 (Reheis), AACH-324 (Summit), AACH-7171 (Summit), Aloxicoll P (Giulini), Aloxicoll SD100
- Aluminiumsesquichlorhydrat [Al₂(OH)_{4,5}Cl_{1,5}] x H₂O
   ∘ Standard Al-Komplexe: Aluminum Sesquichlorohydrate (Reheis), AACH-308 (Summit)
   ∘ Aktivierte Al-Komplexe: Reach 301 (Reheis)
- Aluminiumdichlorhydrat [Al₂(OH)₄Cl₂] x H₂O

Erfindungsgemäße Aluminium/Zirkoniumsalze sind:
- Aluminium/Zirkonium Trichlorhydrex Glycin [Al₄Zr(OH)₁₃Cl₃] x H₂O x Gly
   ∘ Standard Al/Zr-Komplexe: Rezal 33GP (Reheis), AZG-7164 (Summit), Zirkonal P3G (Giulini)
   ∘ Aktivierte Al/Zr-Komplexe: Reach AZZ 902 (Reheis), AAZG-7160 (Summit), Zirkonal AP3G (Giulini)
- Aluminium/Zirkonium Tetrachlorhydrex Glycin [Al₄Zr(OH)₁₂Cl₄] x H₂O x Gly
   ∘ Standard Al/Zr-Komplexe: Rezal 36G (Reheis), AZG-368 (Summit), Zirkonal L435G (Giulini)
   ∘ Aktivierte Al/Zr-Komplexe: Reach 908 (Reheis), AAZG-7167 (Summit), Zirkonal AP4G (Giulini)
- Aluminium/Zirkonium Pentachlorhydrex Glycin [Al₈Zr(OH)₂₃Cl₅] x H₂O x Gly
- Aluminium/Zirkonium Octachlorhydrex Glycin [Al₈Zr(OH)₂₀Cl₈] x H₂O x Gly

Erfindungsgemäß bevorzugt ist der Einsatz von Aluminiumchlorhydrat (ACH). Dabei ist es vorteilhaft, wenn neben ACH keine weiteren antitranspirant wirkenden, aluminiumhaltigen Wirkstoffe enthalten sind und ACH aus wässriger Lösung in die kosmetischen Zubereitungen der Erfindung eingearbeitet wird.

In einer weiteren vorteilhaften Ausführungsform der Erfindung sind als ein oder mehreren antitranspirant wirkenden Wirkstoffe Piperidiniumsalze der Formel (I) 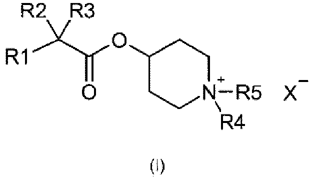 enthalten, wobei Reste R₁ bis R₅ und X⁻ die folgende Bedeutung haben:
- R₄ und R₅: stellen unabhängig voneinander H oder C₁-C₆-Alkyl dar;
- R₁ und R₃: stellen unabhängig voneinander gegebenenfalls substituiertes C₃-C₈-Cycloalkyl, gegebenenfalls substituiertes Phenyl oder gegebenenfalls substituiertes, gesättigtes oder ungesättigtes C₁-C₆-Alkyl dar;
- R₂: stellt H, OH oder OR dar, wobei R C₁-C₇-Alkyl (linear, verzweigt, cyclisch, gesättigt oder ungesättigt, wie z. B. Methyl, Ethyl, n-Propyl, Isopropyl, Allyl, Propargyl, n-Butyl, Cyclopentyl und Cyclohexyl) darstellt; und
- X⁻: stellt ein kosmetisch oder dermatologisch verträgliches Anion dar.

Bevorzugte Piperidiniumsalze sind dabei gewählt aus den 4-[(2-cyclopentyl-2-hydroxy-2-phenylacetyl)oxy]-1,1-dimethyl-piperidiniumsalzen und den 4-[(2-cyclohexyl-2-hydroxy-2-phenylacetyl)oxy]-1,1-dimethyl-piperidiniumsalzen. Insbesondere bevorzugt ist 4-[(2-cyclopentyl-2-hydroxy-2-phenylacetyl)oxy]-1,1-dimethyl-piperidiniumbromid.

Vorteilhaft beträgt der Gesamtanteil der Piperidiniumsalze in der erfindungsgemäßen kosmetischen Zubereitung 0,01 Gew.-% bis 15 Gew.-%, bevorzugt 0,05 Gew.-% bis 10 Gew.-%.

Die erfindungsgemäße kosmetische Zubereitung enthält erfindungsgemäß Wasser. Vorteilhaft beträgt der Anteil an Wasser in der erfindungsgemäßen kosmetischen Zubereitung mindestens 25 Gew.-%, bevorzugt mindestens 30 Gew.-% und insbesondere bevorzugt 32 Gew.-%.

Überraschend hat sich gezeigt, dass vorteilhaft mit zunehmenden Wasseranteil, insbesondere ab einem Wasseranteil von mindestens 32 Gew.-%, beim Anwender ein verbessertes Frischegefühl wahrgenommen wird. Des Weiteren nimmt mit zunehmenden Wasseranteil der an Anteil an öligen Rückständen nach Applikation auf der Haut ab, was vom Verbraucher als vorteilhaft und angenehm empfunden wird.

Schweißgeruch besteht zu einem Großteil aus verzweigtkettigen Fettsäuren, die durch bakterielle Enzyme aus geruchlosem Schweiß freigesetzt werden. Dementsprechend ist es vorteilhaft, wenn die kosmetische Zubereitung zusätzlich ein oder mehrere weitere Wirkstoffe enthält, die der Freisetzung der verzweigtkettigen Fettsäuren entgegen wirken, indem sie das Wachstum und die Vermehrung von Bakterien, vornehmlich von Staphylococcus epidermidis und Corynebacterium jeikeium, reduzieren. Diese Wirkstoffe können erfindungsgemäß ausgewählt werden aus der Gruppe Polyquaternium-16, Polyquaternium-6 und Octenidine.

Im Sinne der Erfindung werden kosmetische Zubereitungen als formstabil bezeichnet, wenn diese in handelsüblicher Form und Größe, definiert über einen Spender, wie bekannt aus dem Produkt Nivea protect&care Antitranspirant (43g Substanz, Mintel Nr. 3570701), der Form entnommen unter den folgenden Bedingungen sowie bei anschließender Akklimatisierung bei Raumtemperatur (21°C, 50% relativer Luftfeuchtigkeit) innerhalb von 24h ihre äußere Form nicht verändern:
1. Lagerung bei -10°C und 50% relativer Luftfeuchtigkeit für 7 Tage
2. Lagerung bei 50°C und 50% relativer Luftfeuchtigkeit für 6 Tage
3. Aufbewahrung im Wechseltemperaturschrank für 6 Tage, wobei die Temperatur alle 12 h zwischen -10°C und +40°C bei 50% relativer Luftfeuchtigkeit alterniert wird und die jeweilige Abkühl- oder Aufheizzeit 5 Minuten bis 2 Stunden beträgt.

Die abschließende Überprüfung erfolgt mit dem bloßen Auge. Jedwede auffallende Veränderung gegenüber der ursprünglichen Form ist als Forminstabilität anzusehen. Als Veränderung der äußeren Form sind auch ein zwischenzeitliches Schmelzen, Brechen oder Rissbildung der kosmetischen Zubereitung zu sehen.

Die erfindungsgemäßen kosmetischen Zubereitungen sind ferner dadurch gekennzeichnet, dass diese fest sind. Im Sinne der vorliegenden Erfindung ist eine kosmetische Zubereitung als fest zu bezeichnen, wenn diese eine Penetrationskraftwert im Bereich von 300 Gramm-Kraft bis 600 Gramm-Kraft, bevorzugt 400 Gramm-Kraft bis 500 Gramm-Kraft aufweist.

Erfindungsgemäß wird der Penetrationskraftwert von kosmetischen Zubereitungen wie folgt bestimmt: Mittels eines TA-XT2i Texture Analyzers der Firma Stable Micro Systems (Godalming, Surrey, UK) wird ein Edelstahlkonus (45°, Modell TA 15) mit einer Vorschubgeschwindigkeit von 2 mm/Sekunde senkrecht (axial) bis zu einer Penetrationstiefe von 5,000 mm in die kosmetischen Zubereitung eingefahren. Dabei wird ermittelt welche Maximalkraft aufgewendet werden muss, um den Edelstahlkonus mit der vorgegebenen Vorschubgeschwindigkeit in die kosmetische Zubereitung 5,000 mm tief eindringen zu lassen. Die aufgewendete Maximalkraft entspricht dem Penetrationskraftwert. Der Penetrationskraftwert wird in Gramm-Kraft angegeben. Ein Penetrationskraftwert von 1 Gramm-Kraft bedeutet, dass die Kraft die eine Masse von 1 g ausübt benutzt werden muss, um den Edelstahlkonus entsprechend der Messparameter in die kosmetische Zubereitung einzufahren. Umso größer der Penetrationskraftwert wird, desto fester ist die Zubereitung. Erfindungsgemäß erfolgt die Messung des Penetrationskraftwerts bei einer Umgebungstemperatur von 20°C bis 27°C und 25% bis 60% relativer Luftfeuchtigkeit. Eine Messung ist ausschließlich valide, wenn die kosmetische Zubereitung nicht bricht. Das beschriebene Messverfahren ist auch in WO 2006012971 A1 vorbeschrieben.

Ein Charakteristikum von Deos ist neben ihrer Antitranspirantwirkung die Parfümierung. Erfindungsgemäße können die beschriebenen Zubereitungen duftneutral (nicht parfümiert) oder bevorzugt parfümiert vorliegen.

Für die vorliegende Erfindung werden Parfümstoffe gewählt aus der Gruppe Hexenol cis 3 (CAS 928-96-1), Coumarin (CAS 91-64-5), Butylphenyl Methylpropional (80-54-6), Orange terpenes (CAS 8028-48-6), Beta ionone (CAS 8013-90-9), Orange oil (CAS 8008-57-9), Terpineol (CAS 8000-41-7), Linalool (CAS 78-70-6), Tetrahydrolinalool (CAS 78-69-3), Triethyl Citrate (CAS 77-93-0), Allylamylglucolat (CAS 67634-00-8), 2-Isobutyl-4-hydroxy-4-methyltetrahydropyran (CAS 63500-71-0), Hexyl salicylate (CAS 6259-76-3), Phenylethyl alcohol (CAS 60-12-8), 3-Methyl-5-phenyl-1-pentanol (CAS 55066-48-3), 2-Acetonapthone-1,2,3,4,5,6,7,8-octahydro-2,3,8,8-tetramethyl (CAS 54464-57-2), Methyl cedryl ketone (CAS 32388-55-9), Methyl dihydrojasmonate (CAS 24851-98-7), 2,6-Dimethyl-7-octen-2-ol (CAS 18479-58-8), Benzyl acetate (CAS 140-11-4), Methyl-alpha-ionone (CAS 1335-46-2), Heliotropin (Piperonal, CAS 120-57-0), Benzyl salicylate (CAS 118-58-1), Linalyl acetate (CAS 115-95-7), Geraniol (CAS 106-24-1), Citronellol (CAS 106-22-9), Omega-Pentadecalactone (CAS 106-02-5), Ethylene brassylate (105-95-3), Ethyllinalool (CAS 10339-55-6) und Hexyl cinnamal (CAS 101-86-0).

Die kosmetischen Zubereitungen gemäß der Erfindung können ferner kosmetische Hilfsstoffe und Wirkstoffe enthalten, wie sie üblicherweise in solchen Zubereitungen verwendet werden, z.B. Wirkstoffe, Konservierungsmittel, Konservierungshelfer, Lipide, Substanzen zum Verhindern des Schäumens, Farbstoffe und Farbpigmente, Verdickungsmittel, anfeuchtende und/oder feuchthaltende Substanzen oder andere übliche Bestandteile einer kosmetischen oder dermatologischen Formulierung wie Polymere, Schaumstabilisatoren, organische Lösungsmittel oder Silikonderivate, sofern der Zusatz die geforderten Eigenschaften hinsichtlich der Stabilität nicht beeinträchtigen oder ausgeschlossen sind.

In den nachfolgend aufgeführten Beispielen wird die vorliegende Erfindung beispielhaft beschrieben, ohne dass die Erfindung, deren Anwendungsbreite sich aus der gesamten Beschreibung und den Ansprüchen ergibt, auf die in den Beispielen genannten Ausführungsformen beschränkt sein soll.

Die Zahlenangaben der Beispielrezepturen **1** bis **7** sind Gewichtsanteile bezogen auf die Gesamtasse der Zubereitung, sofern nichts anderes angegeben. Die Anteilsangaben beziehen sich auf den Gewichtsanteil der jeweiligen INCI Substanz.

| **INCI** | **Bsp. 1** | **Bsp. 2** | **Bsp. 3** | **Bsp. 4** | **Bsp. 5** | **Bsp. 6** | **Bsp. 7** |
|---|---|---|---|---|---|---|---|
| Aluminum Chlorohydrate | 15,0 | 15,0 | 15,0 | 20,0 | 15,0 | 17,5 | |
| 4-[(2-cyclopentyl-2-hydroxy-2-phenylacetyl)oxy]-1,1-dimethylpiperidiniumbromid | | | | | | | 1,0 |
| Cetyl Palmitate | 6,5 | 6,0 | 6,0 | 5,5 | 7,0 | 5,8 | 5,8 |
| Hydrogenated Castor Oil | 5,5 | 6,0 | 6,0 | 6,0 | 5,0 | 5,7 | 5,7 |
| PPG-14 Butyl Ether | 6,4 | 5,0 | 5,2 | 7,5 | 7,0 | 5,8 | 5,8 |
| PPG-15 Stearyl Ether | 10,0 | 10,0 | 10,2 | 8,0 | 11,0 | 9,5 | 9,5 |
| C12-15 Alkyl Benzoate | 5,3 | 5,1 | 5,2 | 7,5 | 7,0 | 7,0 | 7,0 |
| Steareth-21 | 0,5 | 0,5 | 0,5 | 0,5 | 0,6 | 0,5 | 0,5 |
| Steareth-2 | 0,5 | 0,5 | 0,5 | 0,5 | 0,5 | 0,5 | 0,5 |
| Oleth-20 | 1,0 | 1,0 | 1,0 | 1,0 | 1,1 | 1,0 | 1,0 |
| **** Palmitic Acid + Stearic Acid + Myristic Acid + Arachidic Acid + Oleic Acid | 4,0 | 3,8 | 3,8 | 3,5 | 3,5 | 4,0 | 4,0 |
| Propylene Glycol | 1,5 | 1,0 | 2,5 | 2,5 | 2,0 | 2,0 | 2,0 |
| Cetearyl Alcohol | | 2,5 | 2,5 | | 3 | 2,6 | 2,6 |
| Cetyl Alcohol | 1,2 | | | 0,8 | | | |
| Stearyl Alcohol | 1,6 | | | 1,7 | | | |
| C20-40 Alkyl Stearate | | 1,0 | | | | | |
| Parfum | q.s. | q.s. | q.s. | q.s. | q.s. | q.s. | q.s. |
| Aqua | ad 100 | ad 100 | ad 100 | ad 100 | ad 100 | ad 100 | ad 100 |

| | | | | | | | |
|---|---|---|---|---|---|---|---|
| ****Rohstoffmischung aus folgenden Carbonsäuren: Palmitic Acid 48,5% Stearic Acid 47% Myristic Acid 2,5% Arachidic Acid 1,5% Oleic Acid 0,5% | | | | | | | |

## Patentansprüche

1. Kosmetische Zubereitung in Form einer O/W-Emulsion, umfassend
a) 0,1 Gew.-% bis 2,5 Gew.-% ein oder mehrere Alkan-1,2-diole mit einer Alkylkette mit 3 bis 5 C-Atomen,
b) zwei oder mehrere Emulgatoren, wobei mindestens ein O/W-Emulgator mit einem HLB-Wert größer 8 und mindestens ein W/O-Emulgator mit einem HLB-Wert bis maximal 8 enthalten ist,
c) ein oder mehrere Fettalkohole, welche ausgewählt sind aus aliphatischen, linearen, primären Alkanolen mit einer Kettenlänge von 6 bis 22 C-Atomen,
d) ein oder mehrere bei 20°C und 1013 hPa flüssige Öle,
e) ein oder mehrere Wachse, die ab einer Temperatur von 40°C bei 1013 hPa ohne Zersetzung schmelzen,
f) eine oder mehrere gesättigte und/oder ungesättigte lineare Carbonsäuren mit einer Kettenlänge von 10 bis 20 C-Atomen,
g) einen oder mehrere antitranspirant wirkende Wirkstoffe, und
h) Wasser,
jeweils bezogen auf das Gesamtgewicht der Zubereitung, wobei die kosmetische Zubereitung einen Penetrationskraftwert von 300 Gramm-Kraft bis 600 Gramm-Kraft aufweist.

2. Kosmetische Zubereitung nach Anspruch 1 **dadurch gekennzeichnet, dass** ein oder mehrere Alkan-1,2-diole mit einer Alkylkette mit 3 bis 5 C-Atomen in einem Gesamtanteil von 0,2 Gew.-% bis 2 Gew.-%, bezogen auf das Gesamtgewicht der Zubereitung, enthalten sind.

3. Kosmetische Zubereitung nach einem der vorhergehenden Ansprüche **dadurch gekennzeichnet, dass** als Alkan-1,2-diol mit einer Alkylkette mit 3 bis 5 C-Atomen Propan-1,2-diol enthalten ist.

4. Kosmetische Zubereitung nach einem der vorhergehenden Ansprüche **dadurch gekennzeichnet, dass** der Gesamtanteil der Emulgatoren 1,5 Gew.-% bis 5 Gew.-%, bevorzugt 2 Gew.-% bis 3 Gew.-%, bezogen auf das Gesamtgewicht der Zubereitung, beträgt.

5. Kosmetische Zubereitung nach einem der vorhergehenden Ansprüche **dadurch gekennzeichnet, dass** als Emulgatoren zwei nicht ionische O/W-Emulgatoren und ein nicht ionischer W/O-Emulgator enthalten sind.

6. Kosmetische Zubereitung nach einem der vorhergehenden Ansprüche **dadurch gekennzeichnet, dass** die jeweiligen Emulgatoren einzeln in einem Anteil von 0,5 Gew.-% bis 1 Gew.-%, bezogen auf das Gesamtgewicht der Zubereitung, enthalten sind.

7. Kosmetische Zubereitung nach einem der vorhergehenden Ansprüche **dadurch gekennzeichnet, dass** als nicht ionische O/W-Emulgatoren Steareth-21 und Oleth-20 gewählt werden.

8. Kosmetische Zubereitung nach einem der Ansprüche 1 bis 6 **dadurch gekennzeichnet, dass** als nicht ionischer W/O-Emulgator Steareth-2 enthalten ist.

9. Kosmetische Zubereitung nach einem der vorhergehenden Ansprüche **dadurch gekennzeichnet, dass** die Fettalkohole in einem Gesamtanteil von 0,5 Gew.-% bis 5 Gew.-%, bevorzugt 0,75 Gew.-% bis 3,1 Gew.-%, bezogen auf das Gesamtgewicht der Zubereitung, enthalten sind.

10. Kosmetische Zubereitung nach einem der vorhergehenden Ansprüche **dadurch gekennzeichnet, dass** als Fettalkohol Cetylalkohol, Stearylalkohol und/oder Mischungen aus diesen gewählt werden.

11. Kosmetische Zubereitung nach einem der vorhergehenden Ansprüche **dadurch gekennzeichnet, dass** der Anteil der ein oder mehreren bei 20°C und 1013 hPa flüssigen Öle 18 Gew.-% bis 27 Gew.-%, bevorzugt 20 Gew.-% bis 25 Gew.-%, insbesondere bevorzugt 21 Gew.-% bis 24 Gew.-%, bezogen auf das Gesamtgewicht der Zubereitung, beträgt.

12. Kosmetische Zubereitung nach einem der vorhergehenden Ansprüche **dadurch gekennzeichnet, dass** als Öle zwei Etheröle und ein Esteröl eingesetzt werden.

13. Kosmetische Zubereitung nach Anspruch 12 **dadurch gekennzeichnet, dass** als Etheröle PPG-14 Butylether und PPG-15 Stearylether gewählt werden.

14. Kosmetische Zubereitung nach Anspruch 12 **dadurch gekennzeichnet, dass** als Esteröl C12-15 Alkylbenzoat gewählt wird.

15. Kosmetische Zubereitung nach Anspruch 12 **dadurch gekennzeichnet, dass** das Gewichtsverhältnis der Etheröle zum Esteröl 4:1 bis 1:1, bevorzugt 3:1 bis 2:1 beträgt.

16. Kosmetische Zubereitung nach einem der vorhergehenden Ansprüche **dadurch gekennzeichnet, dass** der Gesamtanteil der ein oder mehreren Wachsen, die ab einer Temperatur von 40°C bei 1013 hPa ohne Zersetzung schmelzen, in den kosmetischen Zubereitungen 8 Gew.-% bis 15 Gew.-%, bevorzugt 10 Gew.-% bis 13 Gew.-%, insbesondere bevorzugt 11 Gew.-% bis 12 Gew.-%, bezogen auf das Gesamtgewicht der Zubereitung, beträgt.

17. Kosmetische Zubereitung nach einem der vorhergehenden Ansprüche **dadurch gekennzeichnet, dass** als Wachse hydriertes Rizinusöl und Cetylpalmitat gewählt werden.

18. Kosmetische Zubereitung nach einem der vorhergehenden Ansprüche **dadurch gekennzeichnet, dass** die ein oder mehreren gesättigten und/oder ungesättigten linearen Carbonsäuren mit einer Kettenlänge von 10 bis 20 C-Atomen in einem Gesamtanteil von 2,0 Gew.-% bis 5,0 Gew.-% und insbesondere bevorzugt in einem Gesamtanteil von 2,5 Gew.-% bis 4,5 Gew.-%, bezogen auf das Gesamtgewicht der Zubereitung, enthalten sind.

19. Kosmetische Zubereitung nach einem der vorhergehenden Ansprüche **dadurch gekennzeichnet, dass** als antitranspirant wirkende Wirkstoffe ein oder mehrere Aluminium- und/oder Aluminium/Zirkoniumsalze enthalten sind.

20. Kosmetische Zubereitung nach Anspruch 19 **dadurch gekennzeichnet, dass** als antitranspirant wirkender Wirkstoff Aluminiumchlorhydrat enthalten ist.

21. Kosmetische Zubereitung nach Anspruch 19 **dadurch gekennzeichnet, dass** der Anteil der Aluminium- und/oder Aluminium/Zirkoniumsalze 12,5 Gew.-% bis 25 Gew.-%, insbesondere bevorzugt in einem Anteil von 15 Gew.-% bis 20 Gew.-%, bezogen auf das Gesamtgewicht der Zubereitung, beträgt.

22. Kosmetische Zubereitung nach einem der Ansprüche 1 bis 18 **dadurch gekennzeichnet, dass** als ein oder mehreren antitranspirant wirkenden Wirkstoffe Piperidiniumsalze der Formel (I) 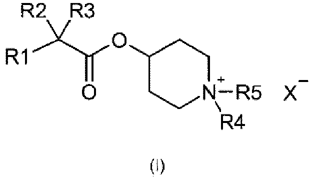 enthalten sind, wobei:
R₄ und R₅ unabhängig voneinander H oder C₁-C₆-Alkyl darstellen;
R₁ und R₃ unabhängig voneinander gegebenenfalls substituiertes C₃-C₈-Cycloalkyl, gegebenenfalls substituiertes Phenyl oder gegebenenfalls substituiertes C₁-C₆-Alkyl darstellen;
R₂ H, OH oder OR darstellt, wobei R C₁-C₇-Alkyl darstellt;
und
X⁻ ein kosmetisch oder dermatologisch verträgliches Anion darstellt.

23. Kosmetische Zubereitung nach Anspruch 22 **dadurch gekennzeichnet, dass** als Piperidiniumsalz 4-[(2-cyclopentyl-2-hydroxy-2-phenylacetyl)oxy]-1,1-dimethyl-piperidiniumbromid enthalten ist.

24. Kosmetische Zubereitung nach einem der Ansprüche 22 bis 23 **dadurch gekennzeichnet, dass** der Gesamtanteil der Piperidiniumsalze 0,01 Gew.-% bis 15 Gew.-%, bevorzugt 0,05 Gew.-% bis 10 Gew.-%, bezogen auf das Gesamtgewicht der Zubereitung, beträgt.

25. Kosmetische Zubereitung nach einem der vorhergehenden Ansprüche **dadurch gekennzeichnet, dass** Wasser in einem Anteil von mindestens 25 Gew.-%, bevorzugt mindestens 30 Gew.-% und insbesondere bevorzugt 32 Gew.-%, bezogen auf das Gesamtgewicht der Zubereitung, enthalten ist.

26. Kosmetische Zubereitung nach einem der vorhergehenden Ansprüche **dadurch gekennzeichnet, dass** zusätzlich Wirkstoffe gewählt aus der Gruppe Polyquaternium-16, Polyquaternium-6 und Octenidine enthalten sind.

27. Verfahren zum Verringern oder Hemmen von Transpiration, wobei das Verfahren das Anwenden einer gegen Transpiration wirksamen Menge der kosmetischen Zubereitung nach einem der Ansprüche 1 bis 26 auf die Haut umfasst.

28. Verwendung der kosmetischen Zubereitung nach einem der Ansprüche 1 bis 26 auf der Haut zur Verringerung von Transpiration.

## Claims

1. Cosmetic preparation in the form of an O/W emulsion, comprising
a) from 0.1% by weight to 2.5% by weight of one or more alkane-1,2-diols with an alkyl chain having from 3 to 5 carbon atoms,
b) two or more emulsifiers, containing at least one O/W emulsifier having an HLB value greater than 8 and at least one W/O emulsifier having an HLB value up to a maximum of 8,
c) one or more fatty alcohols selected from aliphatic, linear, primary alkanols having a chain length of from 6 to 22 carbon atoms,
d) one or more oils liquid at 20°C and 1013 hPa,
e) one or more waxes which melt from a temperature of 40°C at 1013 hPa without decomposition,
f) one or more saturated and/or unsaturated linear carboxylic acids having a chain length of from 10 to 20 carbon atoms,
g) one or more active ingredients with antiperspirant action, and
h) water,
based in each case on the total weight of the preparation, the cosmetic preparation having a penetration force value of from 300 gram-force to 600 gram-force.

2. Cosmetic preparation according to Claim 1, **characterized in that** one or more alkane-1,2-diols with an alkyl chain having from 3 to 5 carbon atoms are present in a total proportion of from 0.2% by weight to 2% by weight, based on the total weight of the preparation.

3. Cosmetic preparation according to either of the preceding claims, **characterized in that** propane-1,2-diol is present as alkane-1,2-diol with an alkyl chain having from 3 to 5 carbon atoms.

4. Cosmetic preparation according to any of the preceding claims, **characterized in that** the total proportion of emulsifiers is from 1.5% by weight to 5% by weight, preferably from 2% by weight to 3% by weight, based on the total weight of the preparation.

5. Cosmetic preparation according to any of the preceding claims, **characterized in that** two nonionic O/W emulsifiers and one nonionic W/O emulsifier are present as emulsifiers.

6. Cosmetic preparation according to any of the preceding claims, **characterized in that** the respective emulsifiers are individually present in a proportion of from 0.5% by weight to 1% by weight, based on the total weight of the preparation.

7. Cosmetic preparation according to any of the preceding claims, **characterized in that** steareth-21 and oleth-20 are selected as nonionic O/W emulsifiers.

8. Cosmetic preparation according to any of Claims 1 to 6, **characterized in that** steareth-2 is present as nonionic W/O emulsifier.

9. Cosmetic preparation according to any of the preceding claims, **characterized in that** the fatty alcohols are present in a total proportion of from 0.5% by weight to 5% by weight, preferably from 0.75% by weight to 3.1% by weight, based on the total weight of the preparation.

10. Cosmetic preparation according to any of the preceding claims, **characterized in that** cetyl alcohol, stearyl alkcohol and/or mixtures thereof are selected as fatty alcohol.

11. Cosmetic preparation according to any of the preceding claims, **characterized in that** the proportion of the one or more oils liquid at 20°C and 1013 hPa is from 18% by weight to 27% by weight, preferably from 20% by weight to 25% by weight, especially preferably from 21% by weight to 24% by weight, based on the total weight of the preparation.

12. Cosmetic preparation according to any of the preceding claims, **characterized in that** two ether oils and one ester oil are used as oils.

13. Cosmetic preparation according to Claim 12, **characterized in that** PPG-14 butyl ether and PPG-15 stearyl ether are selected as ether oils.

14. Cosmetic preparation according to Claim 12, **characterized in that** C12-15 alkyl benzoate is selected as ester oil.

15. Cosmetic preparation according to Claim 12, **characterized in that** the weight ratio of the ether oils to the ester oil is from 4:1 to 1:1, preferably from 3:1 to 2:1.

16. Cosmetic preparation according to any of the preceding claims, **characterized in that** the total proportion of the one or more waxes which melt from a temperature of 40°C at 1013 hPa without decomposition in the cosmetic preparations is from 8% by weight to 15% by weight, preferably from 10% by weight to 13% by weight, especially preferably from 11% by weight to 12% by weight, based on the total weight of the preparation.

17. Cosmetic preparation according to any of the preceding claims, **characterized in that** hydrogenated castor oil and cetyl palmitate are selected as waxes.

18. Cosmetic preparation according to any of the preceding claims, **characterized in that** the one or more saturated and/or unsaturated linear carboxylic acids having a chain length of from 10 to 20 carbon atoms are present in a total proportion of from 2.0% by weight to 5.0% by weight and especially preferably in a total proportion of from 2.5% by weight to 4.5% by weight, based on the total weight of the preparation.

19. Cosmetic preparation according to any of the preceding claims, **characterized in that** one or more aluminum salts and/or aluminum/zirconium salts are present as active ingredients with antiperspirant action.

20. Cosmetic preparation according to Claim 19, **characterized in that** aluminum chlorohydrate is present as active ingredient with antiperspirant action.

21. Cosmetic preparation according to Claim 19, **characterized in that** the proportion of aluminum salts and/or aluminum/zirconium salts is from 12.5% by weight to 25% by weight, especially preferably in a proportion of from 15% by weight to 20% by weight, based on the total weight of the preparation.

22. Cosmetic preparation according to any of Claims 1 to 18, **characterized in that** piperidinium salts of the formula (I) 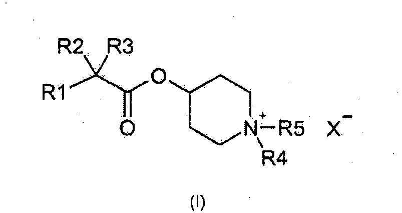 are present as one or more active ingredients with antiperspirant action, where:
R₄ and R₅ are independently of one another H or C₁-C₆-alkyl;
R₁ and R₃ are independently of one another optionally substituted C₃-C₈-cycloalkyl, optionally substituted phenyl or optionally substituted C₁-C₆-alkyl;
R₂ is H, OH or OR, where R is C₁-C₇-alkyl;
and
X⁻ is a cosmetically or dermatologically compatible anion.

23. Cosmetic preparation according to Claim 22, **characterized in that** 4-[(2-cyclopentyl-2-hydroxy-2-phenylacetyl)oxy]-1,1-dimethylpiperidinium bromide is present as piperidinium salt.

24. Cosmetic preparation according to any of Claims 22 to 23, **characterized in that** the total proportion of piperidinium salts is from 0.01% by weight to 15% by weight, preferably from 0.05% by weight to 10% by weight, based on the total weight of the preparation.

25. Cosmetic preparation according to any of the preceding claims, **characterized in that** water is present in a proportion of at least 25% by weight, preferably at least 30% by weight and especially preferably 32% by weight, based on the total weight of the preparation.

26. Cosmetic preparation according to any of the preceding claims, **characterized in that** active ingredients selected from the group consisting of polyquaternium-16, polyquaternium-6 and octenidine are additionally present.

27. Method for decreasing or inhibiting perspiration, the method comprising the application to the skin of a quantity of the cosmetic preparation according to any of Claims 1 to 26 that is effective against perspiration.

28. Use of the cosmetic preparation according to any of Claims 1 to 26 on the skin for decreasing perspiration.

## Revendications

1. Préparation cosmétique sous forme d'une émulsion huile/eau, comprenant
a) 0,1 % en poids à 2,5 % en poids d'un ou plusieurs alcane-1,2-diols dotés d'une chaîne alkyle comportant 3 à 5 atomes de C,
b) deux émulsifiants ou plus, au moins un émulsifiant huile/eau doté d'une valeur de HLB supérieure à 8 et au moins un émulsifiant eau/huile doté d'une valeur de l'HLB d'au maximum 8 étant contenus,
c) un ou plusieurs alcools gras, qui sont choisis parmi des alcanols aliphatiques, linéaires, primaires dotés d'une longueur de chaîne de 6 à 22 atomes de C,
d) une ou plusieurs huiles liquides à 20 °C et 1 013 hPa,
e) une ou plusieurs cires, qui fondent sans décomposition à partir d'une température de 40 °C à 1 013 hPa,
f) un ou plusieurs acides carboxyliques linéaires saturés et/ou insaturés dotés d'une longueur de chaîne de 10 à 20 atomes de C,
g) un ou plusieurs principes actifs à effet antitranspirant, et
h) de l'eau,
à chaque fois par rapport au poids total de la préparation, la préparation cosmétique présentant une valeur de force de pénétration de 300 grammes-force à 600 grammes-force.

2. Préparation cosmétique selon la revendication 1 **caractérisée en ce qu'**un ou plusieurs alcane-1,2-diols dotés d'une chaîne alkyle comportant 3 à 5 atomes de C sont présents en une proportion totale de 0,2 % en poids à 2 % en poids, par rapport au poids total de la préparation.

3. Préparation cosmétique selon l'une quelconque des revendications précédentes, **caractérisée en ce que** le propane-1,2-diol est contenu en tant qu'alcane-1,2-diol doté d'une chaîne alkyle comportant 3 à 5 atomes de C.

4. Préparation cosmétique selon l'une quelconque des revendications précédentes, **caractérisée en ce que** la proportion totale des émulsifiants est de 1,5 % en poids à 5 % en poids, préférablement de 2 % en poids à 3 % en poids, par rapport au poids total de la préparation.

5. Préparation cosmétique selon l'une quelconque des revendications précédentes, **caractérisée en ce que** deux émulsifiants huile/eau non ioniques et un émulsifiant eau/huile non ionique sont contenus en tant qu'émulsifiants.

6. Préparation cosmétique selon l'une quelconque des revendications précédentes, **caractérisée en ce que** les émulsifiants respectifs sont contenus en une proportion de 0,5 % en poids à 1 % en poids, par rapport au poids total de la préparation.

7. Préparation cosmétique selon l'une quelconque des revendications précédentes, **caractérisée en ce que** le stéareth-21 et l'oleth-20 sont choisis en tant qu'émulsifiants huile/eau non ioniques.

8. Préparation cosmétique selon l'une quelconque des revendications 1 à 6, **caractérisée en ce que** le stéareth-2 est contenu en tant qu'émulsifiant eau/huile non ionique.

9. Préparation cosmétique selon l'une quelconque des revendications précédentes, **caractérisée en ce que** les alcools gras sont contenus en une proportion totale de 0,5 % en poids à 5 % en poids, préférablement 0,75 % en poids à 3,1 % en poids, par rapport au poids total de la préparation.

10. Préparation cosmétique selon l'une quelconque des revendications précédentes, **caractérisée en ce que** l'alcool cétylique, l'alcool stéarylique et/ou des mélanges de ceux-ci sont choisis en tant qu'alcool gras.

11. Préparation cosmétique selon l'une quelconque des revendications précédentes, **caractérisée en ce que** la proportion de l'huile ou des huiles liquide(s) à 20 °C et 1 013 hPa est de 18 % en poids à 27 % en poids, préférablement 20 % en poids à 25 % en poids, en particulier préférablement 21 % en poids à 24 % en poids, par rapport au poids total de la préparation.

12. Préparation cosmétique selon l'une quelconque des revendications précédentes, **caractérisée en ce qu'**en tant qu'huiles, deux huiles d'éther et une huile d'ester sont utilisées.

13. Préparation cosmétique selon la revendication 12, **caractérisée en ce qu'**en tant qu'huiles d'éther, l'éther de butyle de PPG-14 et l'éther de stéaryle de PPG-15 sont choisis.

14. Préparation cosmétique selon la revendication 12, **caractérisée en ce qu'**en tant qu'huile d'ester, un benzoate de C₁₂₋₁₅-alkyle est choisi.

15. Préparation cosmétique selon la revendication 12, **caractérisée en ce que** le rapport pondéral des huiles d'éther sur l'huile d'ester est de 4:1 à 1:1, préférablement 3:1 à 2:1.

16. Préparation cosmétique selon l'une quelconque des revendications précédentes, **caractérisée en ce que** la proportion totale de la ou des cires, qui fondent sans décomposition à partir d'une température de 40 °C à 1 013 hPa, dans les préparations cosmétiques est de 8 % en poids à 15 % en poids, préférablement 10 % en poids à 13 % en poids, en particulier préférablement 11 % en poids à 12 % en poids, par rapport au poids total de la préparation.

17. Préparation cosmétique selon l'une quelconque des revendications précédentes, **caractérisée en ce qu'**en tant que cires, de l'huile de ricin hydrogénée et du palmitate de cétyle sont choisies.

18. Préparation cosmétique selon l'une quelconque des revendications précédentes, **caractérisée en ce que** l'acide ou les acides carboxyliques linéaires saturés et/ou insaturés dotés d'une longueur de chaîne de 10 à 20 atomes de C sont contenus en une proportion totale de 2,0 % en poids à 5,0 % en poids et en particulier préférablement en une proportion totale de 2,5 % en poids à 4,5 % en poids, par rapport au poids total de la préparation.

19. Préparation cosmétique selon l'une quelconque des revendications précédentes, **caractérisée en ce qu'**en tant que principes actifs à effet antitranspirant un ou plusieurs sels d'aluminium et/ou d'aluminium/zirconium sont contenus.

20. Préparation cosmétique selon la revendication 19, **caractérisée en ce qu'**en tant que principe actif à effet antitranspirant le chlorhydrate d'aluminium est contenu.

21. Préparation cosmétique selon la revendication 19, **caractérisée en ce que** la proportion des sels d'aluminium et/ou d'aluminium/zirconium est de 12,5 % en poids à 25 % en poids, en particulier préférablement en une proportion de 15 % en poids à 20 % en poids, par rapport au poids total de la préparation.

22. Préparation cosmétique selon l'une quelconque des revendications 1 à 18, **caractérisée en ce qu'**en tant qu'un ou plusieurs principes actifs à effet antitranspirant des sels de pipéridinium de formule (I) 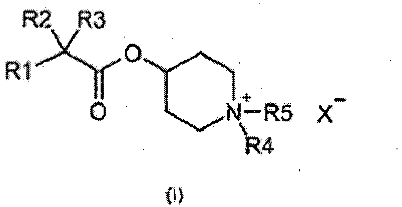 sont contenus,
R₄ et R₅ représentant indépendamment l'un de l'autre H ou C₁₋₆-alkyle ;
R₁ et R₃ représentant indépendamment l'un de l'autre C₃₋₈-cycloalkyle éventuellement substitué, phényle éventuellement substitué ou C₁₋₆-alkyle éventuellement substitué ;
R₂ représentant H, OH ou OR, R représentant C₁₋₇-alkyle ;
et
X⁻ représentant un anion acceptable sur le plan cosmétique ou dermatologique.

23. Préparation cosmétique selon la revendication 22, **caractérisée en ce qu'**en tant que sel de pipéridinium, le bromure de 4-[(2-cyclopentyl-2-hydroxy-2-phénylacétyl)oxy]-1,1-diméthyl-pipéridinium est contenu.

24. Préparation cosmétique selon l'une quelconque des revendications 22 et 23, **caractérisée en ce que** la proportion totale des sels de pipéridinium est de 0,01 % en poids à 15 % en poids, préférablement de 0,05 % en poids à 10 % en poids, par rapport au poids total de la préparation.

25. Préparation cosmétique selon l'une quelconque des revendications précédentes, **caractérisée en ce que** l'eau est contenue en une proportion d'au moins 25 % en poids, préférablement d'au moins 30 % en poids et en particulier préférablement de 32 % en poids, par rapport au poids total de la préparation.

26. Préparation cosmétique selon l'une quelconque des revendications précédentes, **caractérisée en ce que** de plus des principes actifs choisis dans le groupe du polyquaternium-16, du polyquaternium-6 et de l'octénidine sont contenus.

27. Procédé pour la diminution ou l'inhibition de la transpiration, le procédé comprenant l'application d'une quantité efficace contre la transpiration de la préparation cosmétique selon l'une quelconque des revendications 1 à 26 sur la peau.

28. Utilisation de la préparation cosmétique selon l'une quelconque des revendications 1 à 26 sur la peau pour la diminution de la transpiration.
